Europäisches Patentamt

European Patent Office

Office européen des brevets

(19)

(11) Publication number:

**0 275 856**

**A1**

# (12) EUROPEAN PATENT APPLICATION

(21) Application number: 87870176.2

(22) Date of filing: 11.12.87

(51) Int. Cl.⁴: **C12N 15/00** , **C12N 9/72** , **C12N 5/00** , **A61K 37/54**

(30) Priority: 16.12.86 US 942127

(43) Date of publication of application:
27.07.88 Bulletin 88/30

(84) Designated Contracting States:
AT BE CH DE ES FR GB GR IT LI LU NL SE

(71) Applicant: **Smith Kline - RIT Société anonyme dite:**
**rue du Tilleul, 13**
**B-1320 Genval (Rixensart)(BE)**

Applicant: **Leuven Research & Development V.Z.W.**
**Benedenstraat 59A Groot Begijnhof**
**B-3000 Leuven(BE)**

(72) Inventor: **Bollen, Alex Joseph**
**Gaasbeekstraat 65**
**B-1711 Itterbeek(BE)**
Inventor: **Gheysen, Dirk**
**Paardebeekstraat 14**
**B-1900 Overijse(BE)**
Inventor: **Jacobs, Paul**
**Chaussée de la Justice, 173**
**B-7806 Lanquesaint(BE)**
Inventor: **Pierard, Laurent**
**Avenue de Homborchveld, 54**
**B-1180 Bruxelles(BE)**
Inventor: **Collen, Désiré J.**
**Schoonzichtlaan, 20**
**B-3009 Winksele(BE)**

(74) Representative: **Tasset, Gérard**
**SMITHKLINE - RIT rue de l'Institut, 89**
**B-1330 Rixensart(BE)**

(54) **New plasminogen activators.**

(57) New plasminogen activators comprise a fusion of urokinase and tissue plasminogen activator.

EP 0 275 856 A1

## NEW PLASMINOGEN ACTIVATORS

### FIELD OF THE INVENTION

This invention relates to plasminogen activators and to recombinant DNA molecules having coding sequences therefor.

### BACKGROUND OF THE INVENTION

Fibrinolysis is the process whereby intra-and extravascular deposits of fibrin are removed, following activation of a specific fibrinolytic system. It consists of two major events, i.e., plasmin formation and fibrin degradation. In the first event, an inactive precursor, plasminogen, is converted into a proteolytic enzyme, plasmin, plasmin degrades the insoluble fibrin network and hence lyses the clot, plasminogen activators of various sources are known, predominant among these are streptokinase, of bacterial origin, and urokinase (UK) and tissue Plasminogen Activator (tpA). both of human origin.

Biochemical studies on urokinase have shown the existence of three forms of the enzyme. One form has a molecular weight (Mr) of ca. 54,000 daltons and is denominated high Mr-UK (HMW-UK); it consists of two disulfide linked chains A and B, of 18,000 and 33,000 daltons, respectively, The B chain contains the active site of urokinase and is highly homologous to other serine proteases. A second molecular form of urokinase, Mr 33,000 daltons, is derived from HMW-UK by proteolytic cleavage between amino acid 135 and 136 of the A-chain (low Mr UK; LMW-UK) as evidenced from the known amino acid sequence. Both HMWand LMW-UK are good plasminogen activators, used in therapy. Their use as a thrombolytic agent is hampered however by the observed systemic activation of plasminogen which can occur during UK-infusion thereby increasing the risk of haemorrhage.

The existence of a one-chain precursor form of urokinase was demonstrated recently. Upon controlled exposure to plasmin, this precursor is activated to the two-chain HMW-UK. As enzymatically active plasmin, bound to fibrin, is partially protected from inactivation by plasmin inhibitors, such as alpha$_2$-antiplasmin, conversion of the prourokinase to the two-chain molecule could be limited to the surface of the blood clot. Hence, plasminogen activation would be largely restricted to the environment immediately surrounding the fibrin clot by in situ generated HMW-UK. In this respect, therapeutic use of the precursor of HMW-UK could avoid the side effects observed with the active enzyme. Indeed thrombolysis with UK-precursor can be achieved under conditions of very limited systemic activation, in contrast to thrombolysis brought about by UK.

However, it has recently been observed that prourokinase is itself a potent plasminogen activator. The third form of UK, therefore, is single-chain urokinase-type plasminogen activator (scu-PA). The limited systemic activation observed during scu-pA induced thrombolysis, would thus not be caused by fibrin associated conversion of scu-PA to HMW-UK, but rather by direct plasminogen activation by scu-PA, at the fibrin surface. Indeed, due to the presence of a plasma competitor, scu-PA is unable to activate plasminogen in the plasma phase. However, as soon as fibrin is present, the inhibition is counterbalanced and plasmin is formed.

Tissue plasminogen activator is immunologically distinct from urokinase. It is composed of a single polypeptide chain (70,000 daltons) which is converted into a two-chain molecule of comparable activity by proteolytic cleavage. The light chain of tPA, derived from the COOH-terminus of one-chain tPA, contains the active site and is highly homologous to other serine proteases (Pennica et al. Nature 301:214 (1983)); the heavy chain, derived from the NH$_2$-terminus of one-chain tPA, carries structural features similar to those of other proteins in the coagulation and fibrinolytic system. For instance, the heavy chain contains 'kringle'-structures similar to those found in prothrombin, plasminogen and urokinase; it also possesses a growth factor-like domain and another domain, similar to the fibrin binding 'finger'-like structures of fibronectin.

The comparison of HMW-UK and tPA reveals important similarities. The B chain of both molecules, which carries the active sites, are highly homologous and are very similar to other serine protease active sites. On the other hand, the NH$_2$-terminus of both proteins is different. The tPA molecule contains two kringle domains whereas the urokinase species has only one. In addition, tPA carries a fibrin binding function which is absent in urokinase.

## SUMMARY OF THE INVENTION

This invention lies in the discovery that a polypeptide derived from the heavy chain of tPA (chain A) can be fused to a polypeptide from the heavy chain of UK (chain B) to produce a chimaeric, or hybrid, plasminogen activator. In this way novel plasminogen activa tors can be prepared which can have different immunologic or biologic properties over tPA and UK. Preferred such novel plasminogen activators combine the fibrin binding function of tPA with the protease function of urokinase.

Particular embodiments of the invention are plasminogen activators selected from the group consisting of Fg. tPA/UK410, Fg. tPA/UK410/366, tPPUK410/366 and UK-K2410/366.

## BRIEF DESCRIPTION OF THE FIGURES

Figure 1 illustrates recombinant DNA molecules of the invention, showing sequences derived from UK and sequences derived from tPA.

Figure 2 is an updated representation of the eukaryotic shuttle vector DSP1.1BGH (Pfarr et al. DNA, 4, 461-467, 1985).

## DETAILED DESCRIPTION OF THE INVENTION

Using the recombinant DNA molecules of the invention, mutant and chimaeric plasminogen activators can be produced in eukaryotic cells, or other hosts, e.g., bacteria and yeast, and shown to display biological activity. Natural urokinase and tPA molecules have the coding sequence and amino acid sequence shown in Tables 1a and 1b. below. Note that the Jacobs et al. sequence differs from that reported by others, e.g., Gunzler et al., Hoppe Seyler's Z. Physiol. Chem. 363:1155 (1982) Heyneker, Ep-A-92, 182, and Steffens et al (cite). in having Trp at 131 instead of Cys, Cys at 366 instead of Gly and Val at 410 instead of Ala.

## Table 1a

```
        10        20        30        40        50        60        70        80        90       100       110       120
ATGAGAGCCCTGCTGGCGCGCCTGCTTCTCTGCGTCCTGGTCGTGAGCGACTCCAAAGGCAGCAATGAAGTTCATCAAGTTCCATCGAACTGTGACTGTCTAAATGGAGGAAACATGTGTG
  M  R  A  L  L  A  R  L  L  L  C  V  L  V  V  S  D  S  K  G  S  N  E  L  H  Q  V  P  S  N  C  D  C  L  N  G  G  T  C  V
-20                           -10                            1            N  E  L  H  Q  V  P  S         10                20

       130       140       150       160       170       180       190       200       210       220       230       240
TCCAACAAGTACTTCTCCAACATTCACTGGTGCAACTGCCCAAAGAAATTCGGAGGGCAGCACTGTGAAATAGATAAGTCAAAAACCTGCTATGAGGGGAATGGTCACTTTTACCGAGGA
  S  N  K  Y  F  S  N  I  H  W  C  N  C  P  K  K  F  G  G  Q  H  C  E  I  D  K  S  K  T  C  Y  E  G  N  G  H  F  Y  R  G
                              30                            40                            50                            60

       250       260       270       280       290       300       310       320       330       340       350       360
AAGGCCAGCACTGACACCATGGGCCGGCCCTGCCTGCCCTGGAACTCTGCCACTGTCCTTCAGCAAACGTACCATGCCCACAGATCTGATGGTCTTCAGCTGGGCCTGGGGAAACATAAT
  K  A  S  T  D  T  M  G  R  P  C  L  P  W  N  S  A  T  V  L  Q  Q  T  Y  H  A  H  R  S  D  A  L  Q  L  G  L  G  K  H  N
                              70                            80                            90                           100

       370       380       390       400       410       420       430       440       450       460       470       480
TACTGCAGGAACCCGACAACCGGAGGCGACCCTGGTGCTATGTGCAGGTGGGCCTAAAGCCGCTTGTCCAAGAGTGCATGGTGCATGACTGGGCAGATGGAAAAAAGCCGTCGTCTCCT
  Y  C  R  N  P  D  N  R  R  R  P  W  C  Y  V  Q  V  G  L  K  P  L  V  Q  E  C  M  V  H  D  W  A  D  G  K  K  P  S  S  P
                             110                           120                           130                           140

       490       500       510       520       530       540       550       560       570       580       590       600
CCAGAAGAATTAAAATTTCAGTGTGGCCAAAAGACTCTGAGGCCCCGCTTTAAGATTATTGGGGGAGAATTCACCACCATCGAGAACCAGCCGTGGTTTGCGGCCATCTACAGGAGGCAG
  P  E  E  L  K  F  Q  C  G  Q  K  T  L  R  P  R  F  K  I  I  G  G  E  F  T  T  I  E  N  Q  P  W  F  A  A  I  Y  R  R  H
                             150                           160                           170                           180

       610       620       630       640       650       660       670       680       690       700       710       720
CGGGGGGGCTCTGTCACCTACGTGTGTGGAGGCAGCCTCATCAGCCCTTGCTGGGTGATCAGCGCCACACACTGCTTCATTGATTACCCAAAGAAGGAGGACTACATCGTCTACCTGGGT
  R  G  G  S  V  T  Y  V  C  G  G  S  L  I  S  P  C  W  V  I  S  A  T  H  C  F  I  D  Y  P  K  K  E  D  Y  I  V  Y  L  G
                             190                           200                           210                           220

       730       740       750       760       770       780       790       800       810       820       830       840
CGGCTCAAGGGTTAACTCCAACACGCAAGGGGAGATGAAGTTTGAGGTGGAAAACCTCATCCTACACAAGGACTACAGCGCTGACACGCTTGCTCACCACAACGACATTGCCTTGCTGAAG
  R  L  K  G  N  S  N  T  Q  G  E  M  K  F  E  V  E  N  L  I  L  H  K  D  Y  S  A  D  T  L  A  H  H  N  D  I  A  L  L  K
                             230                           240                           250                           260

       850       860       870       880       890       900       910       920       930       940       950       960
ATCCGTTCCAAGCAGGGCAGGTGTGCGCAGCCATCCCGGACTATACAGACCATCTGCCTGCCCTCGATGTATAACGATCCCCAGTTTGGCACAAGCTGTGAGATCACTGGCTTTGGAAAA
  I  R  S  K  Q  G  R  C  A  Q  P  S  R  T  I  Q  T  I  C  L  P  S  M  Y  N  D  P  Q  F  G  T  S  C  E  I  T  G  F  G  K
                             270                           280                           290                           300

       970       980       990      1000      1010      1020      1030      1040      1050      1060      1070      1080
GAGAATTCTACCGACTATCTCTATCCGGAGCAGCTGAAAAATGACTGTTGTGAAGCTGATTTCCCACCGGGAGTGTCTCAGCCAGCCCCACTACTACGGCTCTGAAGTCACCACCAAAATGCTG
  E  N  S  T  D  Y  L  Y  P  E  Q  L  K  N  T  V  V  K  L  I  S  H  R  E  C  Q  Q  P  H  Y  Y  G  S  E  V  T  T  K  M  L
                             310                           320                           330                           340

      1090      1100      1110      1120      1130      1140      1150      1160      1170      1180      1190      1200
TGTGCTGCTGACCCACAGTGGAAAACAGATTCCTGCCAGGGAGACTCAGGGGGACCCCTCGTCTGTTCCCTCCAATGCCGCATGACTTTGACTGGAATTGTGAGCTGGGGCCGTGGATGT
  C  A  A  D  P  Q  W  K  T  D  S  C  Q  G  D  S  G  G  P  L  V  C  S  L  Q  C  R  M  T  L  T  G  I  V  S  W  G  R  G  C
                             350                           360                           370                           380

      1210      1220      1230      1240      1250      1260      1270      1280      1290      1300      1310      1320
GCCCTGAAGGACAAGCCAGGCGTCTACACGAGAGTCTCACACTTCTTACCCTGGATCCGCAGTCACACCAAGGAAGAGAATGGCCTGGTCCTCTGA
  A  L  K  D  K  P  G  V  Y  T  R  V  S  H  F  L  P  W  I  R  S  H  T  K  E  E  N  G  L  V  L
                             390                           400                           410
```

## Table 1b

```
        10        20        30        40        50        60        70        80        90       100       110       120
ATGGATGCAATGAAGAGAGGGCTCTGCTGTGTGCTGCTGCTGTGTGTGGCAGCAGTCTTCGTTTCGCCCCAGCCAGGAAATCCATGCCCGATTCAGAAGAGGAGGCAGATCTTACCAAGTGATC
 M  D  A  M  K  R  G  L  C  C  V  L  L  L  G  A  V  F  V  S  P  S  Q  E  I  H  A  R  F  R  R  G  A  R  S  Y  Q  V  I
-35           -30                          -20                          -10                                        1

       130       140       150       160       170       180       190       200       210       220       230       240
TGCAGAGATGAAAAAACGCAGATGATATACCAGCAACATCAGTCATGGCTGCGCCCTGTGTCAGAAGCAACGGGTGGAATATTGGTGGTGCAACAGTGGCAGGGCACAGTGGCCAGTCA
 C  R  D  E  K  T  Q  M  I  Y  Q  Q  H  Q  S  W  L  R  P  V  L  R  S  M  R  V  E  Y  C  W  C  M  S  G  R  A  Q  C  H  S
       10                        20                        30                              40

       250       260       270       280       290       300       310       320       330       340       350       360
GTGGCTGTCAAAAGTTGCAGCGAGCCAAGGTGTTTCAACGGGGGCACCTGCCAGCAGGCCCTGTACTTCTCAGATTTCGTGTGCCAGTGCCCCGAAGGATTTGCTGGGAAGTGCTGTGAA
 V  P  V  K  S  C  S  E  P  R  C  F  N  G  G  T  C  Q  Q  A  L  Y  F  S  D  F  V  C  Q  C  P  E  G  F  A  G  K  C  C  E
          50                        60                        70                              80

       370       380       390       400       410       420       430       440       450       460       470       480
ATAGATACCAGGGCCACGTGCTACGAGGACCAGGGCATCAGCTACAGGGGCACGTGGAGCACAGCGGAGAGTGGCGCCGAGTGCACCAACTGGAACAGCAGCGCGTTGGCCCAGAAGCCG
 I  D  T  R  A  T  C  Y  E  D  Q  G  I  S  Y  R  G  T  W  S  T  A  E  S  G  A  E  C  T  N  W  N  S  S  A  L  A  Q  K  P
             90                      100                     110                     120

       490       500       510       520       530       540       550       560       570       580       590       600
TACAGCGGGCGGAGGGCCAGCGCCATCAGGCTGGGCCTGGGGAACCACAACTACTGCAGAAACCCAGATCGGGACTCAAAGCCGTGGTGCTACGTGTTTAAGGCGGGGAAGTACGGCTCA
 Y  S  G  R  R  P  D  A  I  R  L  G  L  G  N  H  N  Y  C  R  N  P  D  R  D  S  K  P  W  C  Y  V  F  K  A  G  K  Y  S  S
            130                     140                     150                     160

       610       620       630       640       650       660       670       680       690       700       710       720
GAGTTCTGCAGCACCCCTGCCTGCTCTGAGGGAAACAGTGACTGCTACTTTGGGAATGGGTCAGCCTACGGTGGCACGCACAGCCTCACCGAGTCGGGTGCCTCGTGCCTCGCCGTGGAAT
 E  F  C  S  T  P  A  C  S  E  G  N  S  D  C  Y  F  G  N  G  S  A  Y  R  G  T  H  S  L  T  E  S  G  A  S  C  L  P  W  N
           170                     180                     190                     200

       730       740       750       760       770       780       790       800       810       820       830       840
TCCATGATCCTGATAGGGAAGGTTTACACAGCACAGAACCCCAGTGCCCAGGCACTGGGGCTGGGCAAACATAATTACTGCCGGAATCCTGATGGGGATGCCAAGCCGTGGTGCCACGTG
 S  M  I  L  I  G  K  V  Y  T  A  Q  N  P  S  A  Q  A  L  G  L  G  K  H  N  Y  C  R  N  P  D  G  D  A  K  P  W  C  H  V
           210                     220                     230                     240

       850       860       870       880       890       900       910       920       930       940       950       960
CTGAAGAACCGCAGGCTGACGTGGGAGTACTGTGATGTGCCCTCCTGCTCCACCTGCGGGCCTGAGACAGTACAGCCAGCCTCAGTTTCGCATCAAAGGGGGCTGTTGCCCGACATCGCC
 L  K  N  R  R  L  T  W  E  Y  C  D  V  P  S  C  S  T  C  G  L  R  Q  Y  S  Q  P  Q  F  R  I  K  G  G  L  F  A  D  I  A
           250                     260                     270                     280

       970       980       990      1000      1010      1020      1030      1040      1050      1060      1070      1080
TCCCACCCCTGGCAGGCTGCCATCTTTGCGAAGCACAGGAGGTCGCCGCGAGAGCGGTTCGTGTGCGGGGGCATACTGCATCAGCTCGTGCTGGATTGTCTCGTGCGGCCCACTGCTTCCAG
 S  H  P  W  Q  A  A  I  F  A  K  H  R  R  S  P  G  E  R  F  L  C  G  G  I  L  I  S  S  C  W  I  L  S  A  A  H  C  F  Q
           290                     300                     310                     320

      1090      1100      1110      1120      1130      1140      1150      1160      1170      1180      1190      1200
GAGAGGTTTCCGCCCCACCACGTGACGGTGATCTTGGGCAGAACATACCGGGTGGTCCCTGGCGAGGAGGAGCAGAAATTTGAAGTCGAAAAATACATTGTCCATAAGGAATTCGATGAT .
 E  R  F  P  P  H  H  L  T  V  I  L  G  R  T  Y  R  V  V  P  G  E  E  E  Q  K  F  E  V  E  K  Y  I  V  H  K  E  F  D  D
           330                     340                     350                     360

      1210      1220      1230      1240      1250      1260      1270      1280      1290      1300      1310      1320
GACACTTACGACAATGACATTGCGCTGCTGCAGCTGAAATCGGATTCGTCCCGGTGTGCCCAGGAGAGCAGCGTGGTCCGCACTGTGTGCCTTCCCCGGCGGACCTGCAGCTGCCGGAC
 D  T  Y  D  N  D  I  A  L  L  Q  L  K  S  D  S  S  R  C  A  Q  E  S  S  V  V  R  T  V  C  L  P  P  A  D  L  Q  L  P  D
             370                     380                     390                     400

      1330      1340      1350      1360      1370      1380      1390      1400      1410      1420      1430      1440
TGGACGGAGTGTGAGCTCTCCGGCTACGGGCAAGCATGAGGCCTTGTCTCCTTTCTATTCGGAGCGGCTGAAGGAGGCTCATGTCAGAGTGTACCCATCCAGCCGGTGCACATCACAACAT
 W  T  E  C  E  L  S  G  Y  G  K  H  E  A  L  S  P  F  Y  S  E  R  L  K  E  A  H  V  R  L  Y  P  S  S  R  C  T  S  Q  H
           410                     420                     430                     440

      1450      1460      1470      1480      1490      1500      1510      1520      1530      1540      1550      1560
TTACTTAACAGAACAGTCACCGACAACATGCTGTGTGCTGGAGACAGTCGGAGCGGCGGGCCCCAGGCAAACTTGCACGACGCCTGCCAGGGCGGATTCGGGAGGCCCCCTGGTGTGTCTG
 L  L  N  R  T  V  T  D  N  M  L  C  A  G  D  T  R  S  G  G  P  Q  A  N  L  H  D  A  C  Q  G  D  S  G  G  P  L  V  C  L
           450                     460                     470                     480

      1570      1580      1590      1600      1610      1620      1630      1640      1650      1660      1670      1680
AACGACGGCCGCATGACTTTGGTGGGGCATCATCAGCTGGGGCCTGGGCTGTGGACAGAAGGATGTCCCGGGTGTGTACACAAAGGTTACCAACTACCTAGACTGGATTCGTGACAACATG
 N  D  G  R  M  T  L  V  G  I  I  S  W  G  L  G  C  G  Q  K  D  V  P  G  V  Y  T  K  V  T  N  Y  L  D  W  I  R  D  N  M
           490                     500                     510                     520

      1690
CGAACCGTGA
 R  P
```

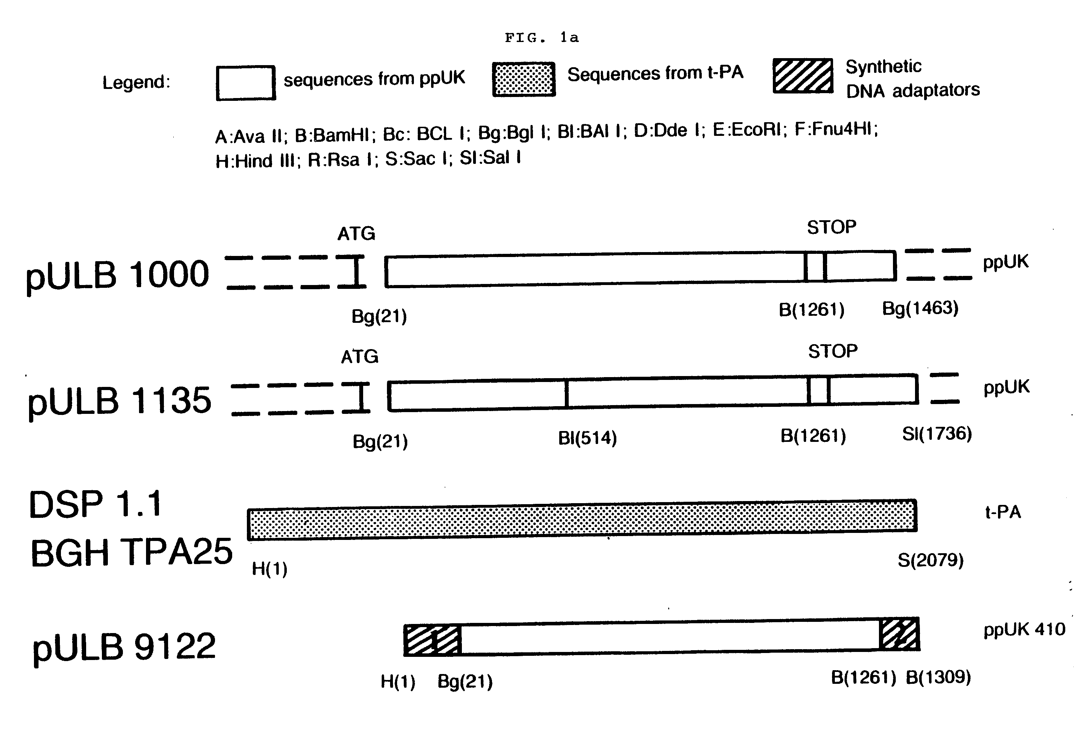

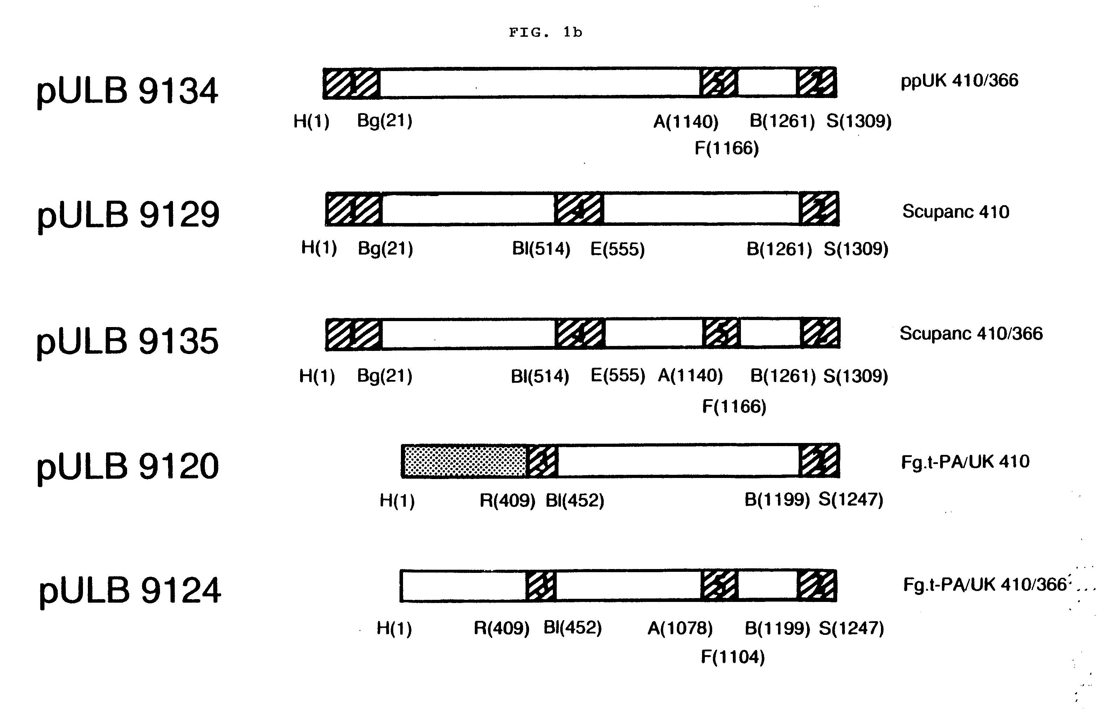

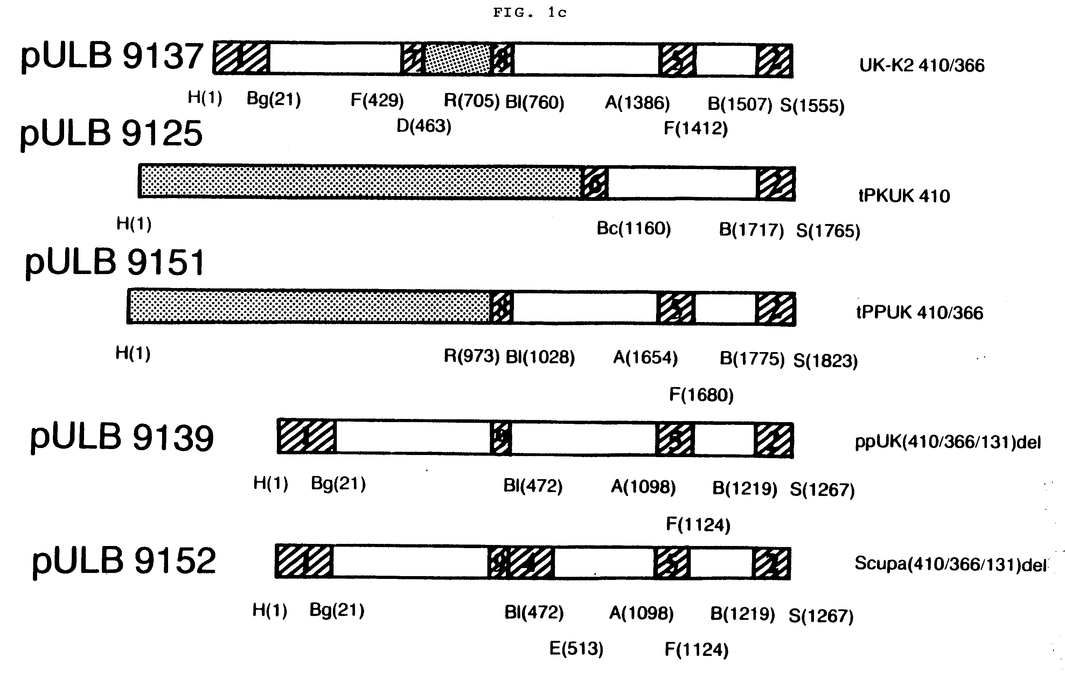

Mutant and chimaeric plasminogen activators, as used herein, have DNA sequences and amino acid sequences different from that of natural urokinase and tPA molecules. A mutant or chimaeric plasminogen activator coding sequence is one which differs from the coding sequence for natural UK and tPA such as is shown in Figure 1. The recombinant DNA molecules of the invention thus, are such mutant or chimaeric coding sequences, either isolated or within a larger DNA molecule such as, for example, a cloning or expression vector. The recombinant molecules of the invention are prepared by standard synthetic or genetic engineering techniques or by a combination of both, as explained herein below.

The coding sequences comprised within the recombinant DNA molecules of this invention combine either one or several DNA sequences derived from UK and tPA DNA molecules or have one or more substitutions, additions or deletions of one or more codons within defined regions of the UK and tPA molecules. The mutant or chimaeric plasminogen activators within exemplary recombinant DNA molecules of the invention, construction and use of which are exemplified herein, are illustrated in Figure 1, as are the natural UK and tPA molecules for comparison, shown in Tables 1a and 1b. The expression and biological activity of mutant or chimaeric plasminogen activators expressed in eukaryotic cells by recombinant DNA molecules of the invention are shown in Table 2, which follows. All of the listed plasmid expression vectors employ the early promoter of SV40 and the Bovine Growth Hormone polyadenylation site.

TABLE 2: Production and biological activities of mutant plasminogen activators.

| Plasmid: | Product: | Production: (ng/ml) | | Biological Activity (plasminogen activation) (IU/mg) | |
|---|---|---|---|---|---|
| | | DSP vector | BPV vector | DSP vector | BPV vector |
| pULB 9122 | ppUK.410 | +++ | +++ | 59200±15000 | ~66600 |
| pULB 9134 | ppUK.410/366 | +++ | +++ | 47300±18000 | |
| pULB 9129 | Scupa nc 410 | ++ | | 3000±1000 | |
| pULB 9135 | Scupa nc 410/366 | +++ | +++ | ~5500 | |
| pULB 9120 | Fg.t-PA/UK.410 | + | +++ | 31300±4000 | ~48800 |
| pULB 9124 | Fg.t-PA/UK.410/366 | + | | 43000±6000 | |
| pULB 9137 | UK-K2.410/366 | + | | 51000±18000 | |
| pULB 9125 | tPKUK.410 | 0 | | - | |
| pULB 9151 | tPPUK.410/366 | + | | 73000±35000 | |
| pULB 9139 | ppUK(410/366/131)del | + | | ~69000 | |
| pULB 9152 | Scupa nc (410/366/131)del | + | | ~2500 | |

LEGENDE: Expression levels: +++ = 45-90 ng/ml; ++ = 21-45 ng/ml; + = 3-18 ng/ml

The plasmid constructions are described in the examples, below. The reported representative expression and biological activity data were obtained by standard techniques (Drapier J.C., Tenu J.P., Lemaire G. and Petit JF Biochemie 61, 463-471, 1979). Figure 1 and Table 2 also show the designation given to the product of each vector.

6

Coding sequences for natural UK and tPA molecules, of which any of the naturally occurring polymorphs can be used as a starting material for carrying out this invention, can be obtained by standard techniques such as those used by Jacobs et al. (1985) for urokinase, and by Pennica et al. (1983) for tPA. See, also, e.g., for tPA: Opdenakker et al., Eur. J. Biochem. 121:269 (1982), Edlund et al., Proc. Natl. Acad. Sci. USA 80:349 (1983); Goeddel et al., EP-A-93.619; Levinson et al., EP-A-117,059, Goldberg et al., PCT WO85-03949; Meyhack et al., EP-A-143,081; and, for UK: Heyneker et al.. EP-A-92,182; Hiramatsu et al., EP-A-154,272; Kovacevic et al., GB 2133797.

The recombinant DNA molecules shown herein are illustrative only. Alternative or additional combinations of DNA fragments or substitutions of base pairs and deletions of codons can be made by techniques well-known in the field of molecular genetics. The effect of such combinations, substitutions and deletions, on expression in eukaryotic cells can be readily determined by an enzyme-linked immunosorbant assay (ELISA) involving monoclonal antibodies as disclosed for example by Herion et al. (1983). Activity of resulting proteins can be readily assayed in well-known screens such as for instance the direct amidolytic activity assay with the chromogenic substrate S2444 (Kabi Vitrum, Stockholm, Sweden) or by an assay in which the catalyzed conversion of plasminogen to plas min is detected by the chromogenic substrate S2251 (Kabi Vitrum, Stockholm, Sweden) (Drapier et al. 1979).

Mutations in the plasminogen activator molecules can be made by well-known techniques. For a review of certain such useful techniques, see Botstein et al. Science (1985). These include, for example, deletion of codons by treatment of the coding sequence with a restriction endonuclease optionally followed by treatment with an exonuclease, e.g. Bal31 or mung bean nuclease. Following such deletion, synthetic sequences can be inserted to replace part or all of the deleted region.

The plasminogen activator coding sequence typically is cloned in a bacterial cell, e.g., E. coli for propagation. Following cloning, the coding sequence is fused in-frame to a regulatory region which functions in a selected host to prepare a gene expression unit. Such host can be any microorganism or cell for which a cloning and expression system is available. These include, e.g., bacteria, such as E. coli, Bacillus and Streptomyces; yeast, such as Saccharomyces and Pichia; and mammalian cells, such as CHO, R1610, NIH3T3 and COS cells.

Useful regulatory regions generally comprise sequences necessary or desirable for RNA polymerase binding and transcription initiation at the ribosome. In the case of bacterial and yeast cells, it is sometimes advantageous to include a transcription terminator downstream of the coding sequence to stabilize expression.

The preferred host for expression of the recombinant DNA molecules of the invention are mammalian cells. In such cells, the regulatory region can be one which is commonly employed in construction of mammalian cell expression vectors, or a region which performs similar functions. Such region typically comprises a promoter region, that is, a region which functions in binding of RNA polymerase and RNA transcription initiation, upstream of the recombinant plasminogen activator coding sequence. Promoters which are commonly employed in mammalian cell expression vectors and which, therefore, are useful in the present invention include the metallothionein promoter, especially the monkey, mouse and human metallothionein promoter; viral promoters such as the Simian Virus 40 (SV40) early promoter or the SV40 late promoter; viral LTR's, such as the LTR of mouse mammary tumor virus (MMTV), the LTR of human T-cell lymphotrophic viruses (HTLV) I, II or III or the LTR of rous sarcoma virus. Such promoters can be derived from genes or cells in which they are naturally present. Such promoters can also be modified to alter their sequence.

Downstream of the recombinant plasminogen activator coding sequence, there is typically a polyadenylation (poly A) region, that is, a region which functions in polyadenylation of RNA transcripts. Such poly A region apparently plays a role in stabilizing transcription. Polyadenylation regions which are commonly employed in mammalian cell expression vectors and which, therefore, are useful in the present invention include the polyadenylation regions of the SV40 early region, bovine growth hormone and human collagen pro-alpha 2(I). Such regions can be derived from the genes in which they are naturally present. Such regions can also be modified to alter their sequence provided that polyadenylation and transcript stabilization functions are not significantly adversely affected.

Other regulatory functions, including splicing sites, transcriptional activators and enhancers, can also be comprised within the gene expression unit or elsewhere within a DNA molecule comprising the gene expression unit.

A mammalian cell is transfected with the recombinant plasminogen activator gene expression unit by any technique that permits introduction of foreign DNA into a host cell without significantly adversely affecting the foreign DNA or the host cell. Such techniques include calcium phosphate precipitation, electroporation, protoplast fusion, microinjection and viral transfection.

The plasminogen activator gene expression unit of the invention preferably is incorporated into a larger DNA molecule prior to transfection. Such larger DNA molecule preferably comprises at least a genetic selection marker in addition to the recombinant plasminogen activator functions. The selection marker can be any gene or genes which cause a readily detectable phenotypic change in a transfected host cell. Such phenotypic change can be, for example, foci formation as can occur when a bovine papilloma virus (BPV) vector is employed; drug resistance, such as genes for neomycin, G418 or hygromycin B resistance; or other selectable markers such as xanthine guanine phosphoribosyl transferase (xgprt), thymidine kinase (TK) and galactokinase (galK). A selection marker which supplies amplification functions can be employed to increase copy number, whether by increased transfection efficiency or by enhanced intra-cellular replication, of the marker and of the gene expression unit. Such markers which also serve to amplify gene copy number include genes for dihydrofolate reductase (methotrexate resistance). CAD (N-phosphonacetyl-L-aspartate resistance) and adenosine deaminase (2-deoxycorformycin resistance).

The larger DNA molecule comprising the recombinant plasminogen activator gene expression unit can be a plasmid which may or may not be capable of episomal maintenance in a mammalian cell transfectant. Such plasmid which is capable of stable, episomal maintenance will comprise, in addition to the tPA gene expression unit, a maintenance function, that is, a region which causes the DNA to replicate independently of mitotic chromosomal replication, in a HAK cell. Such maintenance functions are typically of viral origin, including, for example, animal viruses such as bovine papilloma virus (BFV), Simian Virus 40 (SV40), BK virus (BKV), adenovirus, Epstein Barr Virus (EBV), polyoma viruses and herpes simplex virus (HSV). Such maintenance functions can be readily prepared and tested for stable, episomal maintenance in a mammalian cell. Plasmid, and therefore recombinant plasminogen activator gene, copy number is typically in the range of 1-1000.

Plasmids within which a recombinant plasminogen activator gene expression unit can be incorporated are available from many sources or can be constructed by techniques well-known in the art of gene expression in mammalian hosts by recombinant DNA techniques. Among the sources are public depositories, including the American Type Culture Collection, Rockville, Maryland, which includes several useful plasmids in its catalog.

The gene expression unit can be co-transfected with other DNA functions, such as a gene for an amplifiable function as described above. Such other DNA function can be linked directly or indirectly to the recombinant plasminogen activator gene expression unit or it can be unlinked, that is, on a different molecule. See, for example, Axel, U.S. patent 4,399,216.

Following transfection, a cell which carries the gene expression unit is cultured in a nutrient medium under conditions such that recoverable quantities of recombinant plasminogen activator are produced. Standard mammalian cell culture medium can be employed. Cell cultures are maintained at ambient pressure at 30 to 45°C, preferably at 35 to 42°C and most preferably at 37°C.

A standard protocol includes transformation with plasmid DNA comprising a recombinant plasminogen activator gene expression unit by the calcium precipitation technique followed, after about 4-6 hours incubation, by a glycerol shock treatment. Transformation typically results in the host cell having up to about 1 to 20 copies of the tPA gene expression unit. Amplification techniques can be used to increase that copy number up to about 1000. About 24 hours after transformation, the cells are placed in selective medium and cultured for about 2 to 3 weeks. Surviving colonies are placed in 24 well micro-titer plates and incubated until confluency, i.e., about 1/2 to 1 week. The wells are then assayed for recombinant plasminogen activator production. High-producing colonies are then expanded by successive incubations, each about 1 week, in T25 flasks, T75 flasks, roller bottles and, finally production units. In production units, conditioned medium can be circulated or periodically withdrawn and replaced so that the plasminogen activator can be harvested continuously or periodically. Production units are preferably any culture system designed for adherent cells, cell culture units which employ fibers, microcarriers or microcapsules and cell factories.

Following cell culture, the recombinant plasminogen activator is isolated from conditioned medium by standard protein isolation techniques. These typically involve a series of chromatography procedures including, for example, ion exchange, reverse phase and affinity chromatography procedures. Following purifi cation, the plasminogen activator is formulated into a pharmaceutical composition such that each dosage unit comprises an amount of plasminogen activator which is non-toxic, that is, free of significant adverse side effects, and which is effective in causing thrombolysis following administration to an animal, particularly man. Such pharmaceutical composition for treatment of myocardial infarction typically is in a form suitable for intravenous injection or infusion and comprises 0.1 to 10 mg/ml of the plasminogen activator in a pharmaceutically acceptable diluent or carrier. A typical treatment for an adult person suffering from acute myocardial infarction comprises intravenous infusion of 80-150 mg of the plasminogen activator over a period of .25-6 hours. Suitable diluents and carriers are well-known to a pharmaceutical chemist, as are techniques for preparing the pharmaceutical composition.

EXAMPLES

The examples which follow are illustrative, and not limiting, of the invention. All enzymes were obtained from commercial sources and were used substantially in accordance with the vendors' instructions. DNA adaptors were prepared by DNA synthesis and are shown in Table 3, below.

Table _3_: Sequences of the synthetic DNA adaptors.

**ADAPTOR 1:**

```
                    Met Arg Ala Leu L(eu)

      5' AG CTT ACC ATG AGA GCC CTG C  3'     21-MER
      3'       A TGG TAC TCT CGG GAC G  5'    17-MER

          Hind III                   Bgl I blunt
```

**ADAPTOR 2:**

```
          Ile Arg Ser His Thr Lys Glu Glu Asn Gly Leu Ala Leu ***

      5' G ATC CGC AGT CAC ACC AAG GAA GAG AAT GGC CTG GCC CTC TGA GAG CT 3'  48-MER
      3'       GCG TCA GTG TGG TTC CTT CTC TTA CCG GAC CGG GAG ACT C       5'  40-MER

          BamHI                                                     Sac I
```

**ADAPTOR 3:**

```
      (T)yr Lys Pro Ser Ser Pro Pro Glu Glu Leu Lys Phe Gln Cys Gl(y)

      5' AC AAG CCC TCG AGT CCT CCA GAA GAA TTA AAA TTT CAG TGT GG 3'    43-MER
      3' TG TTC GGG AGC TCA GGA GGT CTT CTT AAT TTT AAA GTC ACA CC 5'    43-MER

          Rsa I                                                  Bal I
```

**ADAPTOR 4:**

```
      (Gl)y Gln Lys Thr Leu Arg Pro Thr Phe Thr Ile Ile Gly Gly G(lu)

      5' C CAA AAG ACT CTG AGG CCC ACC TTT ACC ATT ATT GGG GGA G       3' 41-MER
      3' G GTT TTC TGA GAC TCC GGG TGG AAA TGG TAA TAA CCC CCT CTT AA 5' 45-MER

          Bal I                                              Eco RI
```

**ADAPTOR 5:**

```
      (G)ly Pro Leu Val Cys Ser Leu Gln Gly

      5' GA CCC CTC GTC TGT TCC CTC CAA GGC  3' 26-MER
      3'    GG GAG CAG ACA AGG GAG GTT CCG G 5' 24-MER

          Ava II                    Fnu4H I
```

**ADAPTOR 6:**

```
      (Se)r Pro Cys Trp Va(l)

      5' C CCT TGC TGG GT      3' 12-MER
      3' G GGA ACG ACC CAC TAG 5' 16-MER

                    Bcl I
```

**ADAPTOR 7:**

```
      (P)ro Leu Val Gln Glu Cys Met Val His Asp Cys Ser Glu Gl(y)

      5' CG CTA GTA CAA GAG TGC ATG GTC CAT GAC TGC TCT GAG GG      3' 40-MER
      3'    C GAT CAT GTT CTC ACG TAC CAG GTA CTG ACG AGA CTC CCA GCT 5' 43-MER

      Fnu4H I                                            Sal I
```

**ADAPTOR 8:**

```
      (T)yr Cys Asp Val Pro Ser Cys Ser Ser Pro Pro Glu Glu Leu Lys Phe Gln Cys Gl(y)

      5' AC TGT GAC GTC CCC AGC TGC TCG AGT CCT CCC GAG GAA CTT AAG TTT CAG TGT GG 3' 55-MER
      3' TG ACA CTG CAG GGG TCG ACG AGC TCA GGA GGG CTC CTT GAA TTC AAA GTC ACA CC 3' 55-MER

          Rsa I                                                           BAL I
```

**ADAPTOR 9:**

```
      (P)ro Leu Val Gln Glu Cys Met Val His Asp Cys Ser Thr Cys Gl(y)

      5' CA CTA GTC CAA GAG TGC ATG GTG CAT GAC TGC TCC ACC TGT GG 3' 43-MER
      3'  T GAT CAG GTT CTC ACG TAC CAC GTA CTG ACG AGG TGG ACA CC 5' 42-MER

                                                      Bal I
```

## EX1 pULB 9122

The urokinase gene was isolated from the cDNA clone pULB1000 described in Jacobs et al. (1985).

The gene was obtained on a BglI-BglI (1440 bp) fragment encompassing the UK-cDNA molecule from aa-16 in the signal sequence to the stop codon, the poly C extension and 124 bp of pBR322 sequences. This fragment was treated with T4 DNA polymerase (Maniatis et al. (1982)) and ligated on its 5′ end to a 21 bp HindIII-BglI (blunt ended site) double-stranded synthetic DNA adaptor (adaptor 1, Table 3) to reconstruct the sequence corresponding to the ATG initiation codon and to the urokinase signal peptide up to aa-16.

The reconstructed molecule coding for preprourokinase, ppUK, was then ligated (Maniatis et al. (1982)) to the intermediate E. coli plasmid, pULB1221 (Jacobs et al. (1984)), cut with HindIII and BalI. The resulting recombinant plasmid was further manipulated firstly to remove excedentary 3′ pBR322 sequences, and secondly to substitute the codon for Val410 present in pULB1000 or pULB1135 (Jacobs et al. (1985)) by a codon specifying alanine. This replacement was done in view of the fact that the amino acid sequence reported in Gunzler et al. (1982), Steffens et al. (1982) and Heyneker et al., EPA-92,182 carries an Alanine at position 410.

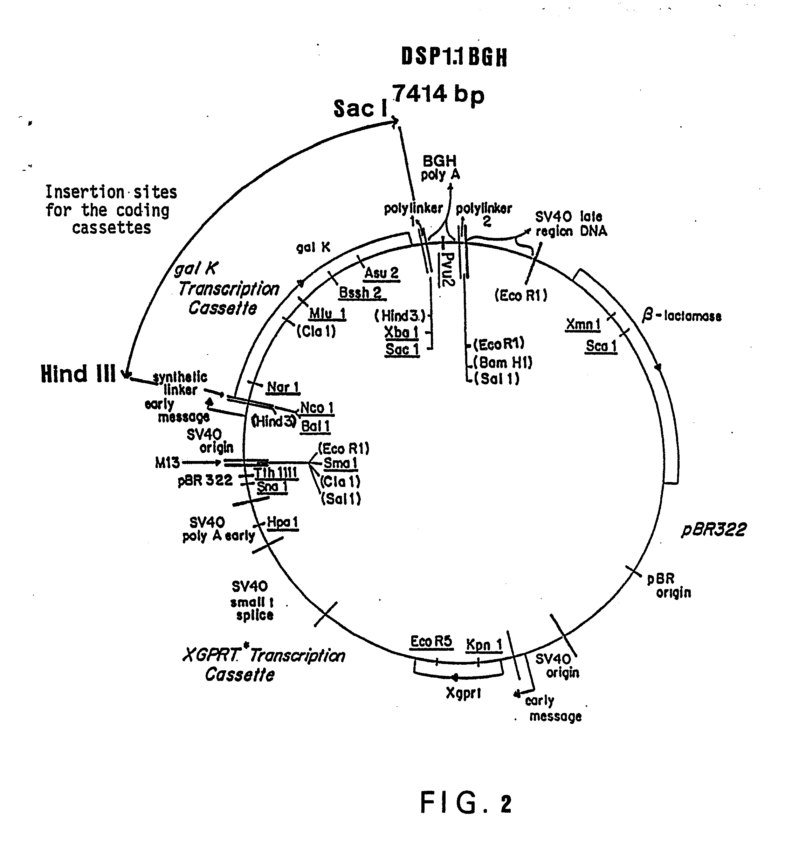

This was done by eliminating from the plasmid a 211 bp BamHI-SacI fragment encompassing the urokinase coding sequence from aa398 to the stop codon and the pBR322 sequences. This fragment was replaced by a 48 bp BamHI-SacI double-stranded synthetic DNA adaptor (adaptor 2, Table 3) which codes for amino acid 398 up to the stop codon which is immediately upstream from the SacI site. The recombinant plasmid deriving from this construction, pULB9117, was introduced by trans formation into E. coli competent cells (Maniatis et al. (1982)). One of the transformants was grown in rich medium containing ampicillin (Maniatis et al. (1982)) in order to amplify the recombinant plasmid. The 1309 bp HindIII-SacI cassette coding for ppUK410 (Fig. 1, ppUK410) was excised from the plasmid pULB9117 by digestion with the appropriate enzymes and inserted by ligation into the eukaryotic shuttle vector DSP1.1BGH (Pfarr et al. DNA, 4, 461-467. 1985). See Fig. 2.

The resulting hybrid plasmid, pULB9122, was introduced into E. coli competent cells and amplified. It was then used to transfect eukaryotic R1610 hamster and Cos1 cells. Conditions were as follows: 20 ug of pure pULB9122 plasmid DNA, twice purified by CsCl gradient centrifugation was added to F80 cm2 dishes with approximately $2 \times 10^6$ cells in Dulbecco's modified minimal essential medium (DMEM) supplemented with 5% fetal calf serum via the calcium phosphate coprecipitation procedure described in Wigler et al. (1979) and Pfarr et al. (1985). After 4 hours of incubation cells were shocked with 10% glycerol in DMEM for 4 min. After 3-5 days, the cell supernatant was recovered to assay the production level and the biological activity of the product coded for by pULB9122. This was done by measuring the amount of ppUK410 in the medium by an ELISA technique using monoclonal antibodies (AAU2 and AAU6) raised against urokinase (Herion et al. (1983)) and by measuring the potential activation of plasminogen by ppUK410 in the medium using a chromogenic substrate (S 2251) (Drapier et al., 1979). Data are shown in Table 2.

Subsequently, the DNA sequence coding for ppUK410 and the flanking SV40 promoter and BGH polyA was introduced as a SalI expression cassette into another eukaryotic vector derived from BPV. This BPV-derived sta ble expression vector carries a unique SalI-site and the mouse cDNA DHFR gene apart from sequences to shuttle in E. coli. The recombinant plasmid (Mini BPV-DHFR-ppUK410) was used to transform HAK cells by the calcium phosphate co-precipitation technique as described above. Individual colonies were obtained 11-14 days later by direct selection of these transfected cells in medium containing elevated levels (200-400 nM) of methotrexate. These colonies were subsequently isolated on day 16-18 by cloning cylinders, further propagated, expanded and screened for UK-activity by the direct S2444 assay and the presence of related UK-antigen was monitored by ELISA as described.

## EX2 pULB9120

A cDNA coding sequence which had been prepared by reverse transcription of mRNA from HeLa cells (Schleuning et al., Sixth Int'l. Conf. Fibrinolysis, Lausanne, Switzerland, July 22-23, 1982) was inserted in-frame into DSP1.1BGH between the SV40 early protein promoter and a BGH polyadenylation site (Woychik et al., Biochem. 81:3944 (1984)); Goodwin et al., EP-A-173,552) to prepare a tPA gene expression unit. The tPA gene expression unit was bounded on both ends by a SalI restriction endonuclease cleavage site in the plasmid, DSP1.1TPA25BGH.

A 409 bp DNA fragment was derived by digestion with the enzymes HindIII and Rsal from the tPA clone. This fragment encompasses the sequences corresponding to the ATG initiation triplet, the signal sequence, the so-called finger module and part of the so-called EGF module (up to aa67) of the tPA molecule (Pennica et al., 1983).

On the other hand, a 1219 bp DNA fragment was derived from the urokinase cDNA, clone pULB1135 (Jacobs et al., 1985) by digestion with the enzymes Ball and Sall. This fragment encompasses the cDNA molecule from aa149 in the proteolytic moiety of urokinase up to the stop codon and includes 3' pBR322 sequences.

A 43 bp double stranded synthetic DNA adaptor carrying Rsal and Ball ends, encoding amino acid 67 of tPA and 136 to 149 of prourokinase (adaptor 3, Table 3) was prepared by chemical synthesis techniques wellknown in the art. The three fragments described above were then ligated together and to the E. coli intermediate vector (pULB1221, Jacobs et al., 1984) DNA previously cut with HindIII and Sall. The resulting recombinant plasmid, introduced into competent E. coli MM294 cells and amplified, was further manipulated to remove extraneous 3' pBR322 sequences. This was done as described in example 1. The final recombinant molecule, pULB9115. was used to transform E. coli competent cells and amplified. The 1247 bp HindIII-Sacl cassette coding for Fg.tPA/UK410 (Figure 1) was excised from the plasmid pULB9115 by digestion with the appropriate enzymes and inserted by ligation into the eukaryotic shuttle vector DSp1.1BGH (Pfarr et al., 1985). The resulting recombinant plasmid, pULB9120, was amplified and then used to transfect eukaryotic R1610 hamster cells as shown in example 1. Further manipulations, such as the assay for the production of Fg.tPA/UK410 and the measure of biological activity were done as in example 1. Data are shown in Table 2. Subsequent introduction of the DNA sequence coding for Fg.tPA/ UK410 into the other eukaryotic expression vector BPV, for obtaining a permanent cell line expressing Fg.tPA/ UK410 hybrid molecule, was done as in example 1. But here, depending on the used BPV-vector type, G418 resistant colonies (400 ug/ml Geneticin Gibco) were now selected. Subsequent isolation, propagation, expanding and scree ning for biological activity and UK-antigen related expression was done as in example 1. Testing production and biological activity in the growing media of the transfected cells with this recombinant plasmid were done as in example 1. Data are shown in Table 2.

## EX3 pULB9129

This construction is a recombinant plasmid carrying the coding sequence for preprourokinase modified in such a way that the natural activation site (Arg156 Phe157 Lys158) is altered (Thr156 Phe157 Thr158) in order to prevent proteolytic activation of the molecule in this site.

Starting from plasmid pULB9117 (see example 1), a 3175 bp Ball-BamH1 DNA fragment was recovered following digestion with the appropriate enzymes. This fragment carries on its 3' end, the ATG initiation signal. the sequence coding from aa-19 to aa149 of preprourokinase and, on its 5' end, the sequence coding for aa398 to the stop signal, those two regions being separated by the sequences of pULB1221 present in the plasmid. On the other hand, a 292 bp EcoRI-Xholl DNA fragment was derived from pULB9117; it codes for aa163 up to aa260 of preprourokinase. A third fragment needed for the construction was prepared also from pULB9117; it is a 414 bp Xholl-BamH1 piece which encodes aa260 to aa398 of the preprourokinase molecule.

At last, a double stranded 41 bp Ball-EcoRI synthetic DNA adaptor coding for aa149 to aa163 including the two amino acid substitutions described above (adaptor 4, Table 3) was prepared by well-known chemical synthesis techniques. The four fragments described above were then ligated together and the resulting recombinant plasmid, pULB9128, was introduced into competent MM294 E. coli cells. After amplification of one of the transformants and purification of the plasmid pULB9128, the HindIII-Sacl cassette coding for the product Scupa nc 410 (Fig. 1) was recovered as a 1309 bp fragment by digestion with appropriate enzymes and introduced by ligation into the eukaryotic shuttle vector DSp1.1BGH (Pfarr et al., 1985). The resulting recombinant plasmid, pULB9129, was used to transform E. coli competent cells and amplified.

It was then introduced in R1610 and Cosl cells as in examples 1 and 2. The expression levels and plasminogen activation capability of the secreted product were tested as described in examples 1 and 2.

EX4 pULB9134

This construction is a recombinant plasmid carrying the sequence coding for preprourokinase modified as to substitute the codon for Cys366, present in pULB1000 or pULB1135, by a codon specifying glycine. This replacement was done in view of the fact that the UK amino acid sequence reported in Guntzler et al. (1982) and in Steffens et al. (1982) carries a glycine at position 366.

Starting from pULB9117 (see example 1), a 2661 bp HindIII-BamH1 DNA fragment was produced by digestion with the appropriate enzymes. This fragment carries, 5' to 3', the sequence for aa398 to the stop codon of the urokinase DNA and sequences derived from pULB1221. On the other hand, a 1140 bp HindIII-AvaII DNA fragment coding for the ATG initiation signal, the signal peptide and aa1 to aa358 of the urokinase molecule was also excised from pULB9117 (example 1). A third natural DNA fragment needed for the construction was prepared by digestion of pULB9117 with the enzymes Fnu4H1 and BamH1. It is a 95 bp long stretch of DNA encoding aa367 to aa398 of urokinase. At last, a double-stran ded 26 bp long AvaII-Fnu4H1 synthetic DNA fragment coding for aa358 to aa366 including the single amino acid substitution described above, was chemically synthesized by techniques well-known in the art (adaptor 5, Table 3).

The four fragments described above were then ligated together and the resulting recombinant plasmid, pULB9119, was introduced into competent MM294 E. coli cells to be amplified. After purification of pULB9119, the HindIII-SacI cassette coding for ppUK410/366 (Fig. 1) was recovered as a 1309 bp fragment and introduced by ligation into the eukaryotic shuttle vector DSP1.1BGH (Pfarr et al., 1985). The recombinant plasmid, pULB9134, was used to transform E. coli competent cells and amplified.

Finally, the plasmid pULB9134 was then introduced in R1610 and CosI cells as in examples 1 and 2. The expression levels and plasminogen activation capability of the secreted product were tested as described in examples 1 and 2.

EX5 pULB9135

The recombinant plasmid, pULB9135, is identical to pULB9129 (example 3) except that the codon for cysteine 366 has been replaced by a codon for glycine. The procedure used to construct the recombinant plasmid pULB9135 is identical to the one described in example 4. The recombinant DNA herein constructed codes for preprourokinase uncleavable at its natural activation site (scupa nc 410/366) and has been amplified in an intermediate E. coli plasmid (pULB9131) as described in above examples (Fig. 1).

A 1309 bp HindIII-SacI cassette was then excised from pULB9131 and introduced into DSP1.1BGH (Pfarr et al., 1985). The recombinant plasmid pULB9135 was then introduced in E. coli and amplified.

It was finally used to transfect R1610 and CosI cells as in examples 1 and 2 (Table 2). The expression levels and plasminogen activation capability of the secreted product were tested as described in examples 1 and 2. Subsequent introduction of the DNA sequence coding for Scupa nc410/366 onto the other eukaryotic expression vector BPV, for obtaining a permanent cell line expressing Scupa nc410/366 was done as in example 1. Subsequent selection, isolation of colonies, propagation, expanding and screening for biological activity and UK-antigen related expression was done as in example 1.

EX6 pULB9124

The recombinant plasmid, pULB9124, is identical to pULB9120 (see example 2) except that the codon corresponding to aa366 of preprourokinase has been replaced by a codon for glycine in order to have a sequence in accordance with the amino acid sequence previously published by Guntzler et al. (1982) and Steffens et al. (1982). The procedure to construct this plasmid was to ligate together a 452 bp HindIII-BalI fragment purified from pULB9115 (see example 2), encoding for the region from the ATG initiation codon to aa67 of tPA and aa136 to 149 of preprourokinase and a 6975 bp HindIII-BalI fragment from pULB9134 (see example 4), encompassing the large HindIII-SacI fragment of DSP1.1BGH wherein all HindIII-SacI cassettes of the invention are inserted to be expressed and the coding sequence for aa149 to the stop codon of preprourokinase.

The plasmid pULB9124 was then introduced in R1610 cells as in examples 1 and 2. The expression levels and plasminogen activation capability of the secreted product. Fg.tPA/UK410/366, were tested as described in examples 1 and 2 (Figure 1, Table 2).

EX7 pULB9125

This construction is a recombinant plasmid coding for an hybrid molecule made of the NH2-part of tPA (signal sequence and aal to aa313) and of the COOH part of urokinase (aa195 to the stop signal). It has been obtained by ligation of the 6 following fragments:

-a 6180 bp HindIII-SacI DNA piece derived from the plasmid vector DSP1.1BGH. It contains all the necessary sequences to express the recombinant DNA in eukaryotic cells.

-a 822 bp HindIII-EcoRI DNA fragment excised from DSP1.1.TPA25BGH. It contains the sequences coding for the initiation signal, the signal peptide and aa1 to aa204 of the tPA molecule.

-a 326 bp EcoRI-AluI DNA fragment purified from DSp1.1.TpA25BGH and encoding aa205 to aa313 of the tPA molecule.

-a 12 bp double-stranded synthetic DNA adaptor coding for aa195 to aa199 of urokinase (adaptor 6, Table 3) which is blunt-ended on its 5' terminus and has BclI cohesive end on its 3' side.

-a 248 bp BclI-Taq1 DNA fragment purified from pULB9117 (see example 1) and coding for aa199 to aa282 of the preprourokinase molecule.

-a 397 bp Taq1-SacI DNA piece derived from pULB9117 (see example 1) and coding for aa282 to the stop codon of preprourokinase.

In this particular example, the coding cassette for the new molecule, here designated as tpKUK410 (Fig. 1), is comprised as a 1805 bp fragment between unique HindIII and SacI sites in the expression vector DSP1.1BGH (Pfarr et al. 1985). The recombinant plas mid, pULB9125, was then introduced into competent E. coli MM294 cells and amplified.

The plasmid pULB9125 was then introduced in R1610 and CosI cells as in examples 1 and 2. The expression levels and plasminogen activation capability of the secreted product were tested as described in examples 1 and 2 (Table 2).

EX8 pULB9137

The construction described herein consists of a recombinant plasmid coding for an hybrid molecule where the amino acid sequence corresponding to the Kringle 2 of tPA is inserted between the Kringle domain and the protease moiety of preprourokinase. The construction, as outlined hereunder, has been obtained by subcloning several DNA fragments into intermediate E. coli plasmid vectors. For practical reasons, pJRD158 (Davison et al. (1984)) and pJRD184 (Heusterspreute et al. (1985)) were used, although they are not obligatory intermediate vectors to achieve the construction. Firstly, a 164 bp NcoI-Fnu4H1 DNA fragment encoding aa66 to aa121 of preprourokinase was excised from pULB9119 (example 4) and a double stranded 40 bp Fnu4H1-SalI synthetic DNA piece (adaptor 7, Table 3) was prepared by techniques well-known in the art. Adaptor 7 codes, in a row, for aa121 to aa130 of urokinase and for aa173 to aa175 of tPA, this sequence being immediately followed by an additional SalI site. The two fragments described above were then ligated to a 2916 bp SalI-NcoI DNA stretch derived from the E. coli cloning vector pJRD184 (Heusterspreute et al. (1985)). The resulting intermediate recombinant plasmid, pBA1, was introduced into competent MM294 E. coli cells and amplified. Secondly, a 258 bp Pst1-RsaI DNA fragment derived from DSP1.1.TPA25BGH (Schleuning et al., Sixth Int'l. Conf. Fibrinolysis, Lausanne, Switzerland, July 22-23, 1982) and coding for aa169 to aa2S5 of the tPA molecule was recovered by digestion. In addition, a double-stranded 55 bp RsaI-BalI synthetic DNA piece (adaptor 8, Table 3) was prepared. Adaptor 8 codes, in a row, for aa255 to aa262 of tPA and for aa139 to aa149 of urokinase. The two fragments were ligated to a 3436 bp BalI-Pst1 DNA stretch derived from the E. coli cloning vector pJRD158 (Davison et al. (1984)).

The resulting intermediate recombinant plasmid, pBA2, was then introduced into competent MM294 E. coli cells and amplified. At last, a 198 bp NcoI-DdeI DNA fragment coding for aa66 to aa130 of urokinase and for aa173 to aa174 of tPA was derived from pBA1. In addition, a 297 bp Dde1-BalI DNA piece coding for aa174 to aa255 of tPA and for aa139 to aa149 of urokinase was excised from pBA2. These two fragments were ligated to a 3673 bp NcoI-BalI DNA fragment derived from pULB9119 (example 4). This last piece of DNA carries on its 3' and the sequences for the ATG initiation signal, for the signal peptide and for aa1 to aa66 of preprourokinase. On its 5' end, it codes for aa149 to the stop signal of preprourokinase. Sequences separating these two 3' and 5' regions originate from pULB1221.

The intermediate recombinant plasmid (pULB9136) resulting from the ligation of the above-described fragments was introduced into E. coli MM294 competent cells and amplified. It was then used to derive, by digestion with appropriate enzymes, a 1555 bp HindIII-SacI coding cassette (Figure 1) corresponding to the product called here UK-K2 410/366. This cassette was introduced by ligation into the shuttle eukaryotic expression vector, DSP1.1BGH (Pfarr et al., 1985) and the resulting recombinant plasmid, pULB9137 was ampli fied before being used to transfect eukaryotic cells.

The plasmid pULB9137 was then introduced in R1610 and Cosl cells as in examples 1 and 2. The expression levels and plasminogen activation capability of the secreted product were tested as described in examples 1 and 2.

## EX9 pULB9151

This construction is a recombinant plasmid coding for an hybrid plasminogen, activator comprising a NH2-terminal segment of tPA up to aa262 combined to a COOH terminal part of urokinase, from aa139 to the last aa of the molecule. It differs from pULB9125 (example 7) by the relative sizes of the tPA and urokinase fragments assembled in the plasmid.

The construction involves three DNA pieces. A first 206 bp EcoRI-Ball DNA fragment was excised from pULB9136 (example 8); it provides the sequence joining the Kringle 2 of tPA and the protease moiety of urokinase. A second 822 bp HindIII-EcoRI DNA fragment, coding for the ATG signal, the signal sequence and aa1 to aa204 of tPA, was obtained by digesting the DNA of plasmid DSp1.1.TPA25BGH with the appropriate enzymes. The last fragment was obtained as a 6975 bp HindIII-Ball stretch by digestion of pULB9134 (example 4). This fragment carries the coding sequence from aa149 to the stop signal of urokinase and all the sequences of the eukaryotic vector DSp1.1BGH (Pfarr et al., 1985) from its unique HindIII site to its unique SacI site. The three fragments described above were ligated to obtain the resulting recombinant plasmid, pULB9151 which carries, as a 1823 bp HindIII-SacI coding cassette, the genetic information for the product herein called tPPUK410/366 (Figure 1). Plasmid pULB9151 was introduced into E. coli MM294 competent cells and amplified prior to its use for transfecting eukaryotic R1610 Hamster cells.

The plasmid pUL89151 as then introduced in R1610 and Cosl cells as in examples 1 and 2. The expression levels and plasminogen activation capability of the secreted product were tested as described in examples 1 and 2 (Table 2).

## EX10 pULB9139

The recombinant molecule described hereunder is a double mutant plasminogen activator; it combines the effects of an amino acid substitution and of a deletion of several amino acids. The recombinant plasmid carries the coding sequence for preprourokinase with the following modifications:

a) amino acid Trp131 has been replaced by a cysteine residue. This has been done in view of the fact that the urokinase molecule described by Guntzler et al. (1982) and by Steffens et al. (1982) carries a cysteine at position 131.

b) the amino acid sequence expanding from residue 132 to residue 147 has been deleted from the molecule and replaced by the dipeptide Ser-Thr as it is the case in the tPA molecule.

The construction involves the ligation of three DNA fragments. A first one was excised from pULB9119 (example 4) as a 3673 bp Ball-NcoI DNA piece. It carries on its 3′ end, the ATG initiation signal, the signal sequence and the codons for aa1 to aa66 of preprourokinase. Sequences coding for aa149 to the stop codon of preprourokinase are located on the 5′ end of the fragment.

Both 3′ and 5′ ends are separated by sequences derived from pULB1221. The second DNA fragment was derived from pULB9119 (example 4) as a 164 bp NcoI-Fnu4H1 DNA piece. It codes for aa66 to aa121 of preprourokinase. The third fragment is a double stranded 43 bp Fnu4H1-Ball synthetic DNA adaptor (adaptor 9, Table 3) obtained by well-known chemical methods. The three fragments described above were ligated and the resulting intermediate recombinant plasmid pULB9138 was introduced into E. coli MM294 competent cells to be amplified. The 1267 bp HindIII-SacI coding cassette was excised from pULB9138 by digestion with the appropriate enzymes; it codes for the product herein designated as ppUK(410/366/131)-del (Figure 1). The cassette was introduced into the eukaryotic shuttle vector DSP1.1BGH (Pfarr et al., 1985) and the resulting recombinant plasmid, pULB9139, was amplified before being used to transfect eukaryotic R1610 Hamster cells.

The plasmid pULB9139 was then introduced in R1610 and Cosl cells as in examples 1 and 2. The expression levels and plasminogen activation capability of the secreted product were tested as described in examples 1 and 2 (Table 2).

EX11 pULB9152

This example describes the construction of a recombinant plasmid, pULB9152 carrying the coding sequences of a preprourokinase unactivable (aa156 and 158 are Thr instead of Arg and Lys, see EX3) and undegradable (the region from aa132 to 147 has been deleted and replaced by the dipeptide Ser-Thr, see EX10). This construction was performed by ligation of a 472 bp HindIII-Ball fragment excised from pULB9138 (EX10) encoding the region from the ATG initiation codon to aa135 of scupa nc (410/366/131)-del, and of a 6975 bp HindIII-Ball purified from pULB9135 (EX5), encompassing the large HindIII-SacI fragment from DSP1.1BGH (Pfarr et al., 1985) wherein all HindIII-SacI cassettes of the invention are inserted to be expressed and the coding sequence for aa149 to the stop codon of scupa nc 410/366 Figure 1).

The plasmid pULB9152 was then introduced in E. coli, amplified, and finally used to transfect R1610 and CosI cells as in examples 1 and 2. The expression levels and plasminogen activation capability of the secreted product were tested as described in examples 1 and 2 (Table 2).

The preceding description and examples fully describe the invention and the preferred embodiments thereof. The invention is not limited to the constructions specifically described but, rather, encompasses all modifications coming within the scope of the following claims.

**Claims**

1. A plasminogen activator selected from the group consisting of Fg.tPA/UK410, Fg.tPA/UK410/366 and tPPUK410/366 and UK-K2 410/366.

2. A recombinant DNA molecule comprising a coding sequence for a plasminogen activator selected from the group consisting of Fg.tPA/UK410, Fg.tPA/UK410/366 or tPPUK410/366 and UK-K2 410/366.

3. The recombinant DNA molecule of claim 2 which is a mammalian expression vector and in which the plasminogen activator coding sequence is fused in-frame to a regulatory region.

4. A transformed mammalian cell which comprises the recombinant DNA molecule of claim 3.

5. A pharmaceutical composition comprising the plasminogen activator of claim 1 and a pharmaceutically acceptable carrier therefore.

6. A process for preparing a plasminogen activator which comprises culturing transformed mammalian cells of claim 4 and isolating the plasminogen activator produced thereby.

## FIG. 1a

Legend: ☐ sequences from ppUK    ▦ Sequences from t-PA    ▨ Synthetic DNA adaptators

A:Ava II; B:BamHI; Bc: BCL I; Bg:Bgl I; Bl:BAl I; D:Dde I; E:EcoRI; F:Fnu4HI;
H:Hind III; R:Rsa I; S:Sac I; Sl:Sal I

pULB 1000    ATG ... STOP ... ppUK
Bg(21)    B(1261)    Bg(1463)

pULB 1135    ATG ... STOP ... ppUK
Bg(21)    Bl(514)    B(1261)    Sl(1736)

DSP 1.1
BGH TPA25    t-PA
H(1)    S(2079)

pULB 9122    ppUK 410
H(1)    Bg(21)    B(1261)   B(1309)

0 275 856

FIG. 1b

pULB 9134 — ppUK 410/366
H(1)  Bg(21)  A(1140)  B(1261)  S(1309)  F(1166)

pULB 9129 — Scupanc 410
H(1)  Bg(21)  Bl(514)  E(555)  B(1261)  S(1309)

pULB 9135 — Scupanc 410/366
H(1)  Bg(21)  Bl(514)  E(555)  A(1140)  B(1261)  S(1309)  F(1166)

pULB 9120 — Fg.t-PA/UK 410
H(1)  R(409)  Bl(452)  B(1199)  S(1247)

pULB 9124 — Fg.t-PA/UK 410/366
H(1)  R(409)  Bl(452)  A(1078)  B(1199)  S(1247)  F(1104)

0 275 856

FIG. 1c

**pULB 9137** UK-K2 410/366

H(1) Bg(21) F(429) R(705) BI(760) A(1386) B(1507) S(1555)
D(463) F(1412)

**pULB 9125** tPKUK 410

H(1) Bc(1160) B(1717) S(1765)

**pULB 9151** tPPUK 410/366

H(1) R(973) BI(1028) A(1654) B(1775) S(1823)
F(1680)

**pULB 9139** ppUK(410/366/131)del

H(1) Bg(21) BI(472) A(1098) B(1219) S(1267)
F(1124)

**pULB 9152** Scupa(410/366/131)del

H(1) Bg(21) BI(472) A(1098) B(1219) S(1267)
E(513) F(1124)

0 275 856

FIG. 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| P,X | THE JOURNAL OF BIOLOGICAL CHEMISTRY, vol. 262, no. 24, 25th August 1987, pages 11771-11778, The American Society for Biochemistry and Molecular Biology, Inc., Baltimore, MD, US; L. PIERARD et al.: "Mutant and chimeric recombinant plasminogen activators" * Whole document * | 1-4,6 | C 12 N 15/00<br>C 12 N 9/72<br>C 12 N 5/00<br>A 61 K 37/54 |
| P,X | THE JOURNAL OF BIOLOGICAL CHEMISTRY, vol. 262, no. 24, 25th August 1987, pages 11779-11784, The American Society for Biochemistry and Molecular Biology, Inc., Baltimore, MD, US; D. GHEYSEN et al.: "Characterization of a recombinant fusion protein of the finger domain of tissue-type plasminogen activator with a truncated single chain urokinase-type plasminogen activator" * Whole document * | 1-4,6 | |
| P,X | EP-A-0 231 883 (SAGAMI CHEMICAL RESEARCH CENTER) * Claims; figure 3 * | 1,2 | TECHNICAL FIELDS SEARCHED (Int. Cl.4)<br><br>C 12 N |
| A | EP-A-0 155 387 (BEECHAM GROUP PLC) * Claim 8; page 4, lines 19-24 *<br>---                                          -/- | 1-6 | |

The present search report has been drawn up for all claims

| Place of search | Date of completion of the search | Examiner |
|---|---|---|
| THE HAGUE | 15-03-1988 | HUBER-MACK A. |

**European Patent Office**

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| P,X | THE JOURNAL OF BIOLOGICAL CHEMISTRY, vol. 262, no. 22, 5th August 1987, pages 10855-10862, The American Society of Biological Chemists, Inc., Baltimore, MD, US; L. NELLES et al.: "Characterization of a fusion protein consisting of amino acids 1 to 263 of tissue-type plasminogen activator and amino acids 144 to 411 of urokinase-type plasminogen activator" * Abstract; page 10855 * | 1-4,6 | |
| P,Y | BIOLOGICAL ABSTRACTS/RRM, abstract no. 34017897, Philadelphia, US; C.J.M. DE VRIES et al.: "Artificial exon shuffling construction of hybrid complementary DNAs containing domains of tissue-type plasminogen activator t-PA and uronkinase u-PA", & THROMBOSIS AND HAEMOSTASIS (WEST GERMANY) 1987, vol. 58, no. 1, p314 * Abstract * | 1 | |
| P,Y | BIOLOGICAL ABSTRACTS/RRM, abstract no. 34017896, Philadelphia, US; S.G. LEE et al.: "Construction and expression of hybrid plasminogen activators prepared from tissue-plasminogen activator t-PA and urokinase u-PA genes", & THROMBOSIS AND HAEMOSTASIS (WEST GERMANY) 1987, vol. 58, no. 1, p313 * Abstract * | 1 | TECHNICAL FIELDS SEARCHED (Int. Cl.4) |

The present search report has been drawn up for all claims

| Place of search | Date of completion of the search | Examiner |
|---|---|---|
| THE HAGUE | 15-03-1988 | HUBER-MACK A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)